# EUROPÄISCHE PATENTSCHRIFT

(11) **EP 4 289 363 B1**
(45) Veröffentlichungstag und Bekanntmachung des Hinweises auf die Patenterteilung: **29.10.2025**
(21) Anmeldenummer: 22177562.0
(22) Anmeldetag: 07.06.2022
(51) Int. Cl.: A61B 6/04, A61B 6/00, A61B 6/03, A61N 5/10

(54) **VERFAHREN ZUR ERMITTLUNG EINER KOMPENSATIONSINFORMATION ZUR KOMPENSATION EINER LIEGENBIEGUNG IN DER COMPUTERTOMOGRAPHIE, COMPUTERTOMOGRAPHIEEINRICHTUNG, COMPUTERPROGRAMM UND ELEKTRONISCH LESBARER DATENTRÄGER**
METHOD FOR DETERMINING COMPENSATION INFORMATION FOR COMPENSATION OF A DEFLECTION OF A BED IN COMPUTER TOMOGRAPHY, COMPUTER TOMOGRAPHY APPARATUS, COMPUTER PROGRAM AND ELECTRONICALLY READABLE DATA CARRIER
PROCÉDÉ DE DÉTERMINATION DES INFORMATIONS DE COMPENSATION DESTINÉE À LA COMPENSATION D'UNE FLEXION DE COUCHETTE EN TOMOGRAPHIE ASSISTÉE PAR ORDINATEUR, DISPOSITIF DE TOMOGRAPHIE ASSISTÉE PAR ORDINATEUR, PROGRAMME INFORMATIQUE ET SUPPORT DE DONNÉES LISIBLES ÉLECTRONIQUEMENT

(43) Veröffentlichungstag der Anmeldung: 13.12.2023
(73) Patentinhaber: Siemens Healthineers AG, 91301 Forchheim (DE)
(72) Erfinder: Baer-Beck, Matthias, 91080 Spardorf (DE)
(74) Vertreter: Siemens Healthineers Patent Attorneys

(56) Entgegenhaltungen:
- DE-A1- 102014 210 458
- DE-T2- 60 109 806
- US-A- 4 233 507
- US-A1- 2008 031 414
- US-A1- 2008 123 924

## Beschreibung

Die Erfindung betrifft ein Verfahren zur Ermittlung einer Kompensationsinformation zur Kompensation einer Biegung einer Liege eines Objekttischs, insbesondere Patiententischs, einer Computertomographieeinrichtung unter dem Gewicht eines aufzunehmenden Objekts, insbesondere eines Patienten, wobei an einer Aufnahmeposition der Liege ein Zielbereichsbilddatensatz eines Zielbereichs des Untersuchungsobjekts mit der Computertomographieeinrichtung aufgenommen wird. Daneben betrifft die Erfindung eine Computertomographieeinrichtung, ein Computerprogramm und einen elektronisch lesbaren Datenträger.

Interventionen werden, insbesondere im medizinischen Bereich, häufig nach vorangehender Bildaufnahme durchgeführt, um einen Zielbereich der Intervention zu identifizieren und/oder zu lokalisieren. Dies ist insbesondere dann sinnvoll, wenn minimalinvasive Interventionen und/oder Strahlenbehandlung/Strahlentherapie durchgeführt werden sollen, da hierbei der Zielbereich ohne direkte Sichtlinie möglichst genau aufgefunden werden soll. Wird als Bildgebungsmodalität für die Interventionsbildgebung Computertomographie eingesetzt, bleibt zwar das Objekt, insbesondere der Patient, auf derselben Liege desselben Objekttischs, insbesondere Patiententischs, positioniert, allerdings ist es in den meisten Fällen notwendig, dass sich die Aufnahmeposition eines Zielbereichsbilddatensatzes zur Lokalisierung des Zielbereichs der Intervention von der Interventionsposition, an der die Intervention nachfolgend durchgeführt wird, unterscheidet, da innerhalb der Gantry beziehungsweise allgemein einer Patientenöffnung der Computertomographieeinrichtung nicht zweckmäßig interventionell gearbeitet werden kann.

Computertomographieeinrichtungen, die auch für Interventionen eingesetzt werden können, weisen üblicherweise einen Objekttisch, insbesondere Patiententisch, auf, der eine Trägerstruktur, beispielsweise wenigstens einen Tischfuß, umfasst, auf dem in wenigstens einer Richtung, insbesondere einer z-Richtung, verschiebbar die Liege, beispielsweise eine Patientenliege, gelagert ist. Nachdem sich die Trägerstruktur naturgemäß außerhalb der Gantry befindet, wird zur Bildaufnahme die Liege von einer Startposition, in der das Objekt beispielsweise oberhalb der Trägerstruktur positioniert ist, derart verfahren, dass die Aufnahmeposition erreicht ist, bei der der Zielbereich des Objekts innerhalb der Gantry und somit innerhalb des Sichtfelds der Computertomographieeinrichtung befindlich ist. Nachdem die Liege mit dem darauf positionierten Objekt hierbei freitragend in die Objektöffnung, insbesondere Patientenöffnung, der Gantry einragt, kann es aufgrund der Gravitationskraft und der hohen zusätzlichen Masse des Objekts zu einer Biegung der Liege aus der Horizontalen unter dem Gewicht des Objekts kommen.

Wird dann eine Planung auf dem Zielbereichsbilddatensatz durchgeführt, bei der beispielsweise zu behandelnde Positionen beziehungsweise allgemein der Zielbereich bzw. auf diesen bezogene Orte lokalisiert werden, kann die Höhe des Zielbereichs in der Aufnahmeposition entsprechend von der Höhe des Zielbereichs an der Interventionsposition abweichen, da eine andere, insbesondere bei Positionierung des Zielbereichs oberhalb der Trägerstruktur überhaupt keine, relevante Biegung der Liege auftritt.

Anders gesagt erfolgt bei Computertomographie-Interventionen üblicherweise zunächst in einem Planungsscan eine Aufnahme eines Zielbereichsbilddatensatzes des Objekts, wonach die Intervention, beispielsweise ein Nadelpfad für eine Biopsie, basierend auf dem Zielbereichsbilddatensatz, insbesondere also den aus dem Planungsscan rekonstruierten Bildern, geplant wird. Aufgrund der Natur der Computertomographie erfolgt der Planungsscan mit dem Objekt innerhalb der Gantry der Computertomographieeinrichtung, wobei jedoch die Intervention, beispielsweise die Biopsie, außerhalb der Gantry der Computertomographieeinrichtung erfolgt. Es liegt also eine Diskrepanz im geometrischen Setting zwischen der Bildaufnahme in der Mitte der Gantry und der Interventionsposition, wo die Intervention durchgeführt wird, vor. Durch die aufgrund des Gewichts des Objekts auftretende Biegung der Liege in vertikaler Richtung werden an der Aufnahmeposition Interventionsbilddaten aufgenommen, die sich an einer niedrigeren Position befinden als der tatsächlichen Position des Zielbereichs während der Intervention.

Werden beispielsweise externe Führungseinrichtungen beziehungsweise Lokalisierungseinrichtungen, beispielsweise laserbasierte Führungseinrichtungen, verwendet, um die den Eingriff durchführende Person während des Eingriffs unterstützend zu führen, beispielsweise durch Visualisierung eines Nadelpfades, tritt ein geometrischer Fehler zwischen dem Zielbereichsbilddatensatz und der realen Objektposition während der Intervention auf, so dass die Intervention gegebenenfalls nicht optimal durchgeführt werden kann, da die Genauigkeit der Visualisierung durch die Führungseinrichtung auf der Annahme basiert, dass sich die geometrischen Beziehungen und Eigenschaften zwischen dem Planungsscan an der Aufnahmeposition und der tatsächlichen Intervention an der Interventionsposition nicht unterscheiden.

Ein anderes Beispiel für derartige, dann nichtinvasive Interventionen ist eine Strahlenbehandlung, insbesondere Strahlentherapie, in einem Zielbereich des Objekts, wo beispielsweise aufgrund des Planungsscans die Behandlungsposition, beispielsweise ein Tumor als Zielbereich, lokalisiert werden kann. Die Lokalisierungsinformation des Zielbereichs wird dann üblicherweise mit Referenzpositionen an der Oberfläche des Objekts, beispielsweise auf der Haut des Patienten, in Beziehung gesetzt. Die Referenzpositionen werden in dem Zielbereichsbilddatensatz markiert und mittels einer beispielsweise laserbasierten Führungseinrichtung durch Visualisierung auf das echte Objekt übertragen, so dass so genannte Bezugsmarker an der Objektoberfläche, beispielsweise der Patientenhaut, vorgesehen werden können, die beispielsweise durch Laser visualisiert werden können.

Auch im Fall der Strahlenbehandlung gilt, dass die Führungseinrichtung außerhalb der Gantry arbeitet, beispielsweise an einer vorderen Abdeckung der Gantry befestigt ist, so dass die Visualisierung außerhalb der Gantry stattfindet. Mithin wird auch hier in unterschiedlichen Positionen geplant und visualisiert. Der Unterschied in der Biegung der Liege überträgt sich dann unmittelbar in einen Fehler hinsichtlich der Positionierung von Bezugsmarkern, was die Qualität der Strahlenbehandlung reduzieren kann.

In diesem Zusammenhang wurde im Stand der Technik bezüglich der Strahlenbehandlung bereits vorgeschlagen, eine Look-up-Tabelle für die Planungsphase beizufügen, die Teil der Dokumentation des Systems ist und die Biegung der Liege bei verschiedenen Positionen anzeigt. Der Nutzer muss dann manuell basierend auf der Look-up-Tabelle den Zielbereichsbilddatensatz beziehungsweise daraus resultierende Ergebnisse korrigieren, was zu Ungenauigkeiten führen kann.

Für die Führung bei einem minimalinvasiven Eingriff als Intervention wurde bereits ein rechnerisches Korrekturvorgehen vorgeschlagen. Dabei wird die Annahme getroffen, dass die Biegung der Liege des Objekttischs durch eine Funktion DT(c, x) beschrieben werden kann, wobei x die Eingabeparameter der Funktion sind, beispielsweise Parameter, von denen die Biegung des Tisches abhängt, und c ein Vektor offener Koeffizienten ist, die zur Berechnung der Tischbiegung auf Basis bekannter Eingangsparameter x kalibriert werden müssen. Dabei kann beispielsweise die Annahme getroffen werden, dass die Biegung des Tisches von der Last auf dem Tisch, der relativen Position der Last auf dem Tisch in Längsrichtung (z-Richtung) und der aktuellen Position des Tisches entlang der Längsrichtung abhängt, beispielsweise relativ zu einer Ruheposition des Tisches. Selbstverständlich sind auch weitere Parameter denkbar, beispielsweise die Ausrichtung des Objekts, bei einem Patienten beispielsweise feet first/head first, die Liegenrichtung, ein Modell des Objekttischs und dergleichen.

Die offenen Koeffizienten c der Funktion DT müssen zuvor kalibriert werden, wobei die Kalibrierung durch Messungen der Tischbiegung bei Verwendung einer definierten und bekannten Last auf der Liege durchgeführt werden. Die offenen Koeffizienten c werden dann so angepasst, dass die Funktion DT die gemessene Liegenbiegung angibt. Beispielsweise können die Koeffizienten durch Minimierung des quadratischen Abstands zwischen der gemessenen Liegenbiegung und der Funktion DT berechnet werden, wobei als Funktion DT beispielsweise ein Polynom herangezogen werden kann.

Bei diesem Vorgehen des Standes der Technik werden üblicherweise Kalibrierungsmessungen für die Liegenbiegung auf einem Referenzsystem, also in einer vergleichbaren Computertomographieeinrichtung, durchgeführt. Die Kalibrierungsmessungen sind äußerst zeitaufwendig und komplex. Hierzu kommt, dass die entsprechende ermittelte Funktion DT nicht für eine bestimmte Computertomographieeinrichtung spezifisch ist, sondern aufgrund der Eigenschaften des Referenzsystems kalibriert ist. Unterschiede zwischen dem Referenzsystem und dem tatsächlich gegebenen System können die Qualität der Abschätzung der Liegenbiegung und der Korrektur negativ beeinflussen.

Dies führt insbesondere auch dazu, dass neue Kalibrierungsmessungen immer dann durchgeführt werden müssen, wenn ein neuer Objekttisch, insbesondere ein neues Modell oder ein neuer Typ, eingeführt wird oder wenn sich die mechanischen Eigenschaften eines gegebenen Typs von Objekttisch ändern. Dies sorgt für hohen Zeitaufwand während dem Entwicklungszyklus. Kalibrierungsmessungen müssen zudem separat für unterschiedliche Konfigurationen des Objekttischs durchgeführt werden. Beispielsweise ist es im Stand der Technik bekannt, eine Zusatzplatte zum Aufbringen auf die Liege zu verwenden, die spezielle, für die Intervention und/oder anderweitig geeignete Eigenschaften aufweist. Derartige Zusatzplatten werden häufig auch als "Overlays" bezeichnet. So ist es beispielsweise für die Strahlentherapie bekannt, steifere Carbonplatten auf die Liege aufzulegen, was selbstverständlich auch einen Einfluss auf die Biegung hat. Dann müssen die Kalibrierungsmessungen für die Funktion DT, die eine Kompensationsinformation zur Kompensation des Höhenunterschieds, also der Biegung der Liege, liefert, separat für jede Kombination von verfügbaren Objekttischen und Zusatzplatten durchgeführt werden. Dies wird insbesondere dann problematisch, wenn beispielsweise Overlays von Drittanbietern benutzt werden können.

Ferner ist bei diesem Ansatz nachteilhaft, dass Kalibrierungskoeffizienten für jede Kombination von Objekttisch und Zusatzplatte/Overlay bereitgestellt und gespeichert werden müssen.

Auch bei sonstigen Vorgängen kann es nützlich sein, eine solche Kompensation durchzuführen, beispielsweise, wenn eine von einer Aufnahmeposition des Zielbereichsbilddatensatzes unterschiedliche Vorgangsposition eine weitere Aufnahmeposition ist, in der ein Zusatzbilddatensatz aufgenommen wird, dessen Bildinhalte mit dem Zielbereich räumlich in Verbindung gebracht werden soll, bei dem dann aber eine andere Biegung der Liege vorliegt. Daher kann der Begriff des Vorgangs bzw. der Vorgangsposition vorliegend nicht nur Interventionen mit entsprechenden Interventionspositionen betreffen, sondern auch darüberhinausgehende Anwendungsfälle.

Als Stand der Technik sind hierbei die US 2008/123924 A1, US 2008/031414 A1, DE 601 09 806 T2 und DE 10 2014 210458 A1 zu nennen.

Der Erfindung liegt daher die Aufgabe zugrunde, eine Alternative zu bekannten Korrekturmöglichkeiten für sich insbesondere zwischen einer Aufnahmeposition und einer Vorgangsposition unterscheidende Liegenbiegungen durch eine Kompensationsinformation anzugeben.

Zur Lösung dieser Aufgabe sind erfindungsgemäß ein Verfahren mit den Merkmalen des Anspruchs 1, eine Computertomographieeinrichtung mit den Merkmalen des Anspruchs 13, ein Computerprogramm mit den Merkmalen des Anspruchs 14 und ein elektronisch lesbarer Datenträger mit den Merkmalen des Anspruchs 15 vorgesehen. Vorteilhafte Ausgestaltungen ergeben sich aus den Unteransprüchen.

Bei einem Verfahren der eingangs genannten Art ist erfindungsgemäß vorgesehen, dass an oder in der Liege wenigstens ein in der Röntgenbildgebung sichtbarer Marker vorgesehen ist und ein dreidimensionaler Referenzdatensatz der Marker bei nicht beladener Liege bereitgestellt wird, wobei die Kompensationsinformation aus einem Vergleich des Referenzdatensatzes mit dem Zielbereichsbilddatensatz ermittelt wird.

Allgemein gesprochen erlaubt die Erfindung also eine Messung der Liegenbiegung durch Vergleich mit einem Referenzdatensatz, in dem die Liegenbiegung durch das Objekt nicht vorliegt. Die Vergleichsgrundlage bilden sowohl in dem Referenzdatensatz als auch in dem Zielbereichsbilddatensatz lokalisierbare Marker. Der Zielbereichsdatensatz kann dabei insbesondere zur Ermittlung einer Lokalisierungsinformation des Zielbereichs dienen, die Gegenstand der Kompensation sein kann.

Auch die vorliegende Erfindung kann in vorteilhaften Anwendungsfällen von einer Situation ausgehen, in der ein auf einen Zielbereich eines Objekts bezogener Vorgang, insbesondere eine Intervention in dem Zielbereich des Objekts, beispielsweise eines Patienten, an einer Vorgangsposition durchgeführt werden soll. Hierzu werden zunächst an der von der Vorgangsposition unterschiedlichen Aufnahmeposition, an der sich der Zielbereich im Sichtfeld der Computertomographieeinrichtung, insbesondere also in einer Objektöffnung der Gantry, befindet, Zielbereichsbilddaten in einem Planungsscan aufgenommen, welche dann ausgewertet werden sollen, um eine auf den Zielbereich bezogene Lokalisierungsinformation bereitzustellen, die an der Vorgangsposition, insbesondere Interventionsposition, genutzt werden soll.

Konkret im Beispiel eines medizinischen Eingriffs gesprochen wird der Patient vor der Intervention also mit dem Zielbereich in die Gantry gefahren und es wird der Zielbereichsbilddatensatz an der entsprechenden Aufnahmeposition aufgenommen. Sodann wird der Patient in die Interventionsposition (als Vorgangsposition) verbracht, und nach Abschluss der Planung kann die Intervention basierend auf der Lokalisierungsinformation durchgeführt werden. Hierbei kann es jedoch aufgrund einer dem Gewicht des aufzunehmenden Objekts geschuldeten Biegung der Liege in der Aufnahmeposition zu Höhenabweichungen zwischen dem Zielbereich im Zielbereichsbilddatensatz und dem realen Zielbereich an der Vorgangsposition kommen, weshalb die vorliegende Erfindung eine Kompensationsinformation ermittelt, die eben diese durch die Liegenbiegung auftretende Höhenabweichung beschreibt und zur Korrektur verwendet werden soll. Dabei kann die Kompensationsinformation, wie noch genauer beschrieben werden wird, die Liegenbiegung beschreiben, mithin einen Wert einer relativen Höhe angeben, um die der Zielbereichsbilddatensatz beziehungsweise die Lokalisierungsinformation zu verschieben ist, um genauere Kenntnis zur Lage des Zielbereichs an der Vorgangsposition zu erhalten und die Intervention mit hoher Genauigkeit und Qualität durchführen zu können.

Anders gesagt kann vorgesehen sein, dass die Kompensationsinformation zur Kompensation einer in dem Vorgang zu verwendenden, insbesondere anzuzeigenden und/oder anzuzielenden, aus dem Zielbereichsbilddatensatz ermittelten Lokalisierungsinformation des Zielbereichs in der Vorgangsposition und/oder zur Höhenkompensation des Zielbereichsbilddatensatzes selber verwendet wird. Die mittels der Kompensationsinformation korrigierte beziehungsweise aus dem korrigierten Zielbereichsbilddatensatz hergeleitete Lokalisierungsinformation kann beispielsweise zur Ansteuerung einer Führungseinrichtung, insbesondere einer laserbasierten Führungseinrichtung zur Visualisierung von auf den Zielbereich bezogenen Positionen und/oder einer einen Monitor umfassenden Anzeigeeinrichtung zur insbesondere Echtzeit-Anzeige einer Position eines insbesondere medizinischen Interventionsinstruments basierend auf Positionsdaten des Interventionsinstruments, verwendet werden.

Allgemein kann im Rahmen der vorliegenden Erfindung vorgesehen sein, dass die Liege auf einer Trägerstruktur des Objekttisches, insbesondere linear und/oder zwischen der Vorgangsposition und der Aufnahmeposition, verfahrbar gelagert ist, wobei selbstverständlich auch über die Vorgangsposition und die Aufnahmeposition hinausgehende Endpositionen der Verfahrbarkeit gegeben sein können. Dabei befindet sich das Objekt, insbesondere der Zielbereich, in der Vorgangsposition bevorzugt zumindest im Wesentlichen oberhalb der Trägerstruktur, so dass dort keine nennenswerte Biegung der Liege auftritt.

Dabei sei an dieser Stelle noch angemerkt, dass auch eine gewisse, deutlich kleinere Biegung unter dem Eigengewicht der Liege auftreten kann, die jedoch für die hier diskutierten Anwendungsfälle vernachlässigt werden kann. Insbesondere ist die Liegenbiegung durch das Gewicht des Objekts um wenigstens eine Größenordnung größer als die Liegenbiegung aufgrund des Eigengewichts der Liege. Beispielsweise sind Objekttische bekannt, bei denen mit einer Last auf der Liege eine Liegenbiegung, mithin ein Höhenunterschied, in Aufnahmepositionen im Bereich von 6 bis 20 mm auftreten kann, während die Verbiegung unter Eigengewicht lediglich 0,3 bis 0,6 mm betragen würde.

Um die Probleme des Standes der Technik, insbesondere die Notwendigkeit für Kalibrierungsmessungen und den Mangel an für eine bestimmte Computertomographieeinrichtung spezifischer Kompensation, zu überwinden, wird erfindungsgemäß ein auf wenigstens einem Marker basierendes Vorgehen zur Messung der Liegenbiegung vorgeschlagen. Dabei wird wenigstens ein Marker, insbesondere mehrere Marker, in die Liege des Objekttischs integriert beziehungsweise positionsfest mit der Liege verbunden. Die Marker sind in Röntgenbildern sichtbar, so dass dies auch für den ohnehin aufzunehmenden Zielbereichsbilddatensatz gilt. Ist nun aus einem für einen Vergleich geeigneten Referenzdatensatz, der erfindungsgemäß ja bereitgestellt wird, bekannt, wo sich die Marker in den Zielbereichsbilddaten befinden sollten, kann durch einen entsprechenden Vergleich die Abweichung, die durch die Biegung der Liege unter dem Gewicht des Objekts auftritt, und somit die Kompensationsinformation ermittelt werden.

Im Vergleich zu den bekannten Herangehensweisen des Standes der Technik werden keine aufwendigen Kalibrierungsmessungen benötigt, sondern nur ein einzelner, Information zur Markerposition ohne Biegung durch ein Objekt enthaltender Referenzdatensatz, der insbesondere für eine bestimmte Computertomographieeinrichtung oder Tischkonfiguration spezifisch sein kann, ist ausreichend. Beispielsweise kann der Referenzdatensatz in einer Referenzmessung bei der Installation der Computertomographieeinrichtung beziehungsweise im Rahmen einer ersten Inbetriebnahme ermittelt werden, wobei eine einfache, nicht lang andauernde und unkomplizierte Wiederholung durch den Betreiber selbst oder einen Service-Ingenieur erfolgen kann, wenn Veränderungen an der Computertomographieeinrichtung vorgenommen werden, die den Objekttisch, insbesondere dessen Liege, betreffen.

In einem anderen Beispiel ist es auch denkbar, den Referenzdatensatz aus der bekannten Konstruktion des Objekttisches und insbesondere der Liege herzuleiten. In jedem Fall besteht keine Notwendigkeit zum Speichern von Kalibrierungsdaten und Tabellen für verschiedene Kombinationen von Objekttischen und Zusatzplatten, insbesondere Overlays. Mit anderen Worten kann auch gesagt werden, dass bei einer mit einer Zusatzlatte, insbesondere austauschbaren Overlays, versehbaren Liege für alle Zusatzplatten derselbe Referenzdatensatz bereitgestellt wird. Denn es ist letztlich irrelevant, ob die Liege durch die Zusatzplatte steifer wird beziehungsweise das Objekt hierdurch höher gelagert ist, da ja lediglich auf eine Referenz Bezug genommen wird, in der eine Biegung durch das Gewicht des Objekts nicht auftritt.

Wie bereits erwähnt, kann eine besonders vorteilhafte Ausgestaltung der vorliegenden Erfindung vorsehen, dass der Referenzdatensatz in einer, insbesondere bei einer erstmaligen Inbetriebnahme der Computertomographieeinrichtung mit aktuellen Liegeneigenschaften durchgeführten, Referenzmessung bei nicht beladener Liege ermittelt wird. Mithin kann das vorgeschlagene Verfahren mit einer Referenzmessung beginnen, bei der kein Patient oder sonstiges Objekt auf der Liege befindlich ist, so dass die Referenzmessung, die wie ein üblicher Computertomographiescan mit der Computertomographieeinrichtung durchgeführt wird, lediglich Bilddaten von dem wenigstens einen Marker und dem Tisch selber, insbesondere der Liege, enthält. Aus den Ergebnissen der Referenzmessung können mittels üblicher Rekonstruktionsverfahren Referenzbilder rekonstruiert werden, in denen dann lediglich der wenigstens eine Marker und die Liege sichtbar sind. Diese Referenzbilder, die aus in der Referenzmessung aufgenommenen Projektionsbildern beispielsweise mittels Rückprojektion rekonstruiert werden können, können den Referenzdatensatz bilden beziehungsweise zu dessen Ermittlung noch weiterverarbeitet werden. Die Referenzmessung muss hinsichtlich einer bestimmten Liegenkonfiguration nur einmal durchgeführt werden. Es sei angemerkt, dass bei einer Änderung der Liegeneigenschaften, beispielsweise bei einem Ersetzen der Liege oder des gesamten Objekttischs, eine erneute Referenzmessung durchgeführt werden kann.

In einer besonders vorteilhaften Ausgestaltung der Erfindung kann vorgesehen sein, dass für die Referenzmessung der in ihrer Höhe verstellbaren Liege eine Referenzhöheneinstellung gewählt wird, so dass der wenigstens eine Marker in jedem Projektionsbild der Referenzmessung erfasst wird, wobei der Referenzdatensatz für die Ermittlung der Kompensationsinformation bei einer Abweichung der Höheneinstellung von der Referenzhöheneinstellung um diese Abweichung in Höhenrichtung verschoben wird. Um ein möglichst genaues Bild von dem wenigstens einen Marker zu erhalten, sollte die Liege so im Sichtfeld der Computertomographieeinrichtung positioniert werden, dass der wenigstens eine Marker idealerweise in allen Projektionsbildern, die in der Referenzmessung aufgenommen werden, sichtbar ist. Bei einer höhenverstellbaren Liege, wobei insbesondere entsprechende Höhenverstellmittel in der Trägerstruktur vorgesehen sein können, kann also zweckmäßigerweise eine relativ hohe Position als Referenzhöheneinstellung gewählt werden, beispielsweise die Liege dort positioniert werden, wo ansonsten das Objekt, insbesondere dessen Zielbereich, anzuordnen wäre. Nachdem der Objekttisch im Allgemeinen ja mit dem Koordinatensystem der Computertomographieeinrichtung registriert ist, ist es selbstverständlich problemlos möglich, auf andere Höheneinstellungen rückzurechnen, beispielsweise solche, bei denen dann der Zielbereich statt der Liege zentral im Sichtfeld der Computertomographieeinrichtung positioniert wird. Es muss lediglich der Referenzdatensatz um die Abweichung der aktuellen Höheneinstellung von der Referenzhöheneinstellung verschoben werden.

Dabei sei der Vollständigkeit halber an dieser Stelle noch angemerkt, dass im Allgemeinen selbstverständlich die aktuelle Position aufgrund entsprechender Einstellungsmittel / Verstellmittel des Patiententischs bekannt ist, insbesondere auch in der Verfahrrichtung, in der insbesondere zwischen der Vorgangsposition und der Aufnahmeposition gewechselt wird, wobei die Verfahrrichtung allgemein bevorzugt der Längsrichtung der Liege entspricht und/oder die insbesondere parallel zur Rotationsachse einer Röntgenquelle beziehungsweise gesamten Aufnahmeanordnung der Computertomographieeinrichtung ist. Entsprechende Einstellmittel für ein lineares Verfahren von Liegen auf Objekttischen sind im Stand der Technik bereits weitgehend bekannt und müssen vorliegend nicht im Detail dargestellt werden. Beispielsweise kann der Objekttisch eine Steuereinheit aufweisen, die die aktuelle Einstellung des Objekttischs einer Steuereinrichtung der Computertomographieeinrichtung bereitstellt.

Alternativ und/oder bei Zusammenführung von Daten zusätzlich kann im Rahmen der vorliegenden Erfindung auch vorgesehen sein, dass der Referenzdatensatz aus Konstruktionsdaten des Objekttisches bereitgestellt wird. Letztlich ist ja aufgrund der Konstruktion des Objekttisches schon bekannt, wo innerhalb der Liegen beziehungsweise an der Liege der wenigstens eine Marker befestigt ist, so dass hieraus ein dreidimensionaler Datensatz erstellt werden kann, der diese Position im unbeladenen Zustand im dreidimensionalen Raum anzeigt. Ein Vergleich mit Röntgendaten des Zielbereichsbilddatensatzes ist dennoch weiterhin möglich, nachdem ja aus den Konstruktionsdaten auch bekannt ist, welche Eigenschaften die verschiedenen Materialien des Objekttisches, insbesondere aber des wenigstens einen Markers, haben. Nachdem auch bei der Aufnahme von Zielbereichsbilddatensätzen verwendbare Röntgenspektren der Computertomographieeinrichtung bekannt sind, ist es möglich, mit Röntgendaten vergleichbare Referenzdaten auch aus Konstruktionsdaten bereitzustellen.

In Weiterbildung der Erfindung kann vorgesehen sein, dass als Marker wenigstens ein längliches, insbesondere drahtartiges, sich in einer, insbesondere zu einer Rotationsachse einer Röntgenquelle der Computertomographieeinrichtung parallelen und/oder einer Längsrichtung der Liege entsprechenden, Verfahrrichtung der Liege, insbesondere zwischen der Aufnahmeposition und der Vorgangsposition, erstreckendes Markerelement verwendet wird. Hierbei kann es zweckmäßig sein, wenn sich das Markerelement zumindest im Wesentlichen über alle im Sichtfeld der Computertomographieeinrichtung positionierbaren Abschnitte der Liege oder sogar zumindest im Wesentlichen über die gesamte Länge der Liege erstreckt. So ist sichergestellt, dass in allen denkbaren Aufnahmepositionen auch tatsächlich eine Aufnahme des wenigstens einen Markerelements denkbar ist. Durch die Parallelität zur Verfahrrichtung zwischen der Vorgangsposition und der Aufnahmeposition, welche üblicherweise der Längsrichtung der Liege entspricht (z-Richtung), ist sichergestellt, dass sich unabhängig von der tatsächlich gewählten Aufnahmeposition für alle Aufnahmepositionen dasselbe Bild des wenigstens einen Markerelements ergibt, so dass der Vergleich hochgenau durchführbar ist. Die Markerelemente können beispielsweise durch dünne Drähte gegeben werden, welche bevorzugt in die Liege integriert sind, beispielsweise in diese eingegossen sind.

Doch auch in anderen Fällen kann es zweckmäßig sein, den wenigstens einen Marker beziehungsweise das Markerelement innerhalb der Liege zu integrieren, da dann das Markerelement besonders vor Positions- und Orientierungsveränderungen beziehungsweise Beschädigungen geschützt ist. Neben der Möglichkeit, beispielsweise die Platte der Liege um das wenigstens eine Markerelement urzuformen, sind selbstverständlich auch andere Möglichkeiten der Integration denkbar, beispielsweise das Vorsehen von zu verschließenden Nuten in der Liege und dergleichen. Alternativ oder zusätzlich ist selbstverständlich auch eine Befestigung wenigstens eines des wenigstens einen Markerelements an der Liege denkbar, beispielsweise durch entsprechende Befestigungsmittel auf der Oberfläche beziehungsweise in entsprechenden hierfür vorgesehenen Vertiefungen.

Dabei ist es, nachdem der Vergleich hierdurch erleichtert wird, besonders zweckmäßig, mehrere Marker und mithin auch Markerelemente zu verwenden, oder aber Markerelemente eines Querschnitts senkrecht zur Längsrichtung zu wählen, der beispielsweise hinsichtlich der Orientierung Vorteile bietet. Dabei ist an dieser Stelle anzumerken, dass das wenigstens eine Markerelement nicht zwangsläufig zu einer vollständigen dreidimensionalen Orientierungsbestimmung geeignet sein muss, da es um die möglichst genaue Bestimmung einer Höhenlage geht, wofür auch in einer Ebene angeordnete, gleichartige Marker bereits äußerst nützlich sein können.

Eine zweckmäßige Weiterbildung der Erfindung kann vorsehen, dass wenigstens drei parallele Markerelemente verwendet werden, insbesondere eines mittig und zwei, bevorzugt symmetrisch, benachbart hierzu, bevorzugt in Randbereichen der Liege, und/oder auf unterschiedlichen Höhen innerhalb der Liege. Es ist mithin möglich, beispielsweise drahtartige Markerelemente einzusetzen, von denen eines in der Mitte, eines links und eines rechts bezüglich des Querschnitts der Liege angeordnet sind, wobei die äußeren Markerelemente bevorzugt an der linken und der rechten Kante der Liege vorgesehen sein können.

Das wenigstens eine, insbesondere zylindrische Markerelement kann beispielsweise einen Durchmesser von 1 bis 20 mm aufweisen und/oder bevorzugt aus Kunststoff bestehen. Allgemein gesprochen eignen sich Materialien mit einer Röntgenschwächung im Bereich von -750 bis 100 HU (Hounsfield Units) besonders. Derartige Materialien sind in einer Referenzmessung beziehungsweise einem Planungsscan hinreichend deutlich in den Bilddaten erkennbar, schwächen jedoch die Röntgenstrahlung nicht zu stark, so dass das Auftreten von Artefakten, wie es beispielsweise von Metallen bekannt ist, möglichst weitgehend vermieden wird. Mithin können eher schwach schwächende Materialien, insbesondere geeignete Kunststoffe wie ABS (Acrylnitril-Butadien-Styrol-Copolymere) eingesetzt werden.

Vorzugsweise kann der Referenzdatensatz nur den wenigstens einen Marker zeigend ermittelt werden. Im Falle einer Referenzmessung ist es in diesem Zusammenhang beispielsweise möglich, den wenigstens einen Marker, insbesondere unter zusätzlicher Nutzung von Konstruktionsdaten, zu segmentieren, wonach alle anderen Bildbereiche mit dem Schwächungswert, insbesondere HU-Wert, für Luft gefüllt werden, mithin -1000 HU. Auf diese Weise ist ein einfach handhabbarer Referenzdatensatz gegeben, für den Vergleiche auf die relevanten Bildinhalte, insbesondere als Markerelemente ausgebildete Marker, konzentriert werden können und andere, eher komplexe Strukturen zur einfachen und aufwandsarmen Ermittlung der Kompensationsinformation nicht beachtet werden müssen. Besondere Vorteile ergeben sich, wenn, was im Rahmen der vorliegenden Erfindung ohnehin bevorzugt ist, der Vergleich im Projektionsraum stattfindet, da dann Vorwärtsprojektionen zur Ermittlung entsprechender Vergleichsbilder aus dem Referenzdatensatz einfach und robust berechenbar sind, wie auch der Vergleich, insbesondere eine Korrelation, einfacher und aufwandsärmer, aber dennoch robust durchführbar ist.

Erfindungsgemäß ist vorgesehen, dass zum Vergleich des Referenzdatensatzes mit dem Zielbereichsbilddatensatz wenigstens ein Vergleichsbild mit einer Aufnahmegeometrie eines zugeordneten Projektionsbilds des Zielbereichsbilddatensatzes durch Vorwärtsprojektion aus dem Referenzdatensatz ermittelt wird und mit dem zugeordneten Projektionsbild im Projektionsraum verglichen wird, insbesondere für mehrere, bevorzugt alle, wenigstens einen des wenigstens einen Markers zeigenden Projektionsbilder des Zielbereichsbilddatensatzes.

Mit anderen Worten kann zur Ermittlung der Liegenbiegung für einen gegebenen Zielbereichsbilddatensatz eine simulierte Vorwärtsprojektion des wenigstens einen Markers aus dem Referenzdatensatz unter Verwendung der Aufnahmegeometrien des tatsächlichen Planungsscans erfolgen, wobei der Vergleich dann im Projektionsraum mit entsprechenden Projektionsdaten des Planungsscans erfolgt. Dabei wurde erkannt, dass bei der Aufnahme des Zielbereichsbilddatensatzes üblicherweise der Zielbereich des Objekts im Zentrum des Sichtfelds der Computertomographieeinrichtung platziert wird, damit dieser in allen Projektionsbildern, insbesondere unter allen verwendeten Projektionswinkeln, erfasst werden kann.

Dies kann aber dazu führen, dass der wenigstens eine Marker nur in einem Teil dieser Projektionsbilder zu sehen ist, so dass bei einem Versuch auch der Rekonstruktion des wenigstens einen Markers in den Bildraum aus den Projektionsbildern aufgrund mangelnder Daten Fehler und/oder lokaler Artefakte auftreten können, die den Vergleich im Bildraum erschweren könnten. Anders gesagt kann es aufgrund der äußerst niedrigen vertikalen Position der Liege für Vorgänge, insbesondere interventionelle Anwendungen, vorkommen, dass wenigstens ein Teil der Liege nicht immer im Sichtfeld der Computertomographieeinrichtung befindlich ist, so dass es bei Anwendung dieses markerbasierten Ansatzes im Bildraum der Schwächungswerte zu Problemen kommen kann. Durch die Anwendung in der Projektionsdomäne, wie hier vorgeschlagen, wird diese Beschränkung umgangen, da der wenigstens eine Marker in den Projektionsdaten wenigstens teilweise sichtbar ist, insbesondere eher in anterior-posterior-Projektionsbildern beziehungsweise posterior-anterior-Projektionsbildern.

Hierbei kann der Vergleich bei mehrere Zeilen umfassend aufgenommenen Projektionsbildern zeilenweise und/oder nach einer Umrechnung in eine Parallelstrahlgeometrie erfolgen. Besonders bevorzugt ist es jedoch, wenn für unterschiedliche Aufnahmegeometrien ein gemeinsamer Vergleich, beispielsweise die Ermittlung eines gemeinsamen Korrelationswerts, durchgeführt wird, indem beispielsweise die Projektionsbilder als wenigstens ein Projektionsdatensatz, der Projektionsdaten für unterschiedliche Projektionswinkel und/oder Bildkoordinaten enthält, formuliert werden.

Eine besonders zweckmäßige Weiterbildung in diesem Kontext ergibt sich, wenn die Vergleichsbilder und die Projektionsbilder vor dem Vergleich unter Verwendung eines gleichen Filters hochpassgefiltert werden. Die Hochpassfilterung kann sich dann insbesondere auf wenigstens eine Dimension senkrecht zur Erstreckungsrichtung des länglichen, wenigstens einen Markerelements beziehen, wodurch insbesondere in den Projektionsbildern des Zielbereichsbilddatensatzes der wenigstens eine Marker hinreichend deutlich hervorgehoben wird. Konkret kann vorgesehen sein, dass zur Hochpassfilterung ein zweidimensionaler Gaußscher Tiefpassfilter angewendet wird und das Ergebnis vom jeweiligen ungefilterten Bild abgezogen wird.

In bevorzugter Ausgestaltung der Erfindung kann in einer konkreten Herangehensweise zur Ermittlung der Kompensationsinformation vorgesehen sein, dass aus dem Referenzdatensatz unter Berücksichtigung der Aufnahmegeometrie für den Zielbereichsbilddatensatz Vergleichsbilder für unterschiedliche relative Höhen des wenigstens einen Markers ermittelt werden, wobei eine optimierte relative Höhe mit einer höchsten Übereinstimmung des wenigstens einen entsprechenden Vergleichsbilds mit wenigstens einem entsprechenden Zielbereichsbild des Zielbereichsbilddatensatzes als Kompensationsinformation bestimmt wird.

Wie bereits diskutiert wurde, ist der Referenzdatensatz ja zum Koordinatensystem der Computertomographieeinrichtung registriert. Er definiert eine auf den nicht durch ein Objekt belasteten Zustand der Liege bezogene Referenzhöhe, die gegebenenfalls bezüglich einer Abweichung der Höheneinstellung, wie diskutiert, angepasst ist. Die Referenzhöhe der Marker entspricht also der Annahme, dass keine Liegenbiegung durch ein darauf befindliches Objekt bei der aktuell verwendeten Höheneinstellung vorliegt. Die Abweichung von dieser Referenzhöhe ist die zu kompensierende Höhenabweichung, also die relative Höhe, wobei verschiedene mögliche Höhenabweichungen durch Verschiebung des Referenzdatensatzes im Koordinatensystem der Computertomographieeinrichtung beziehungsweise durch Verschieben der Aufnahmegeometrie im Koordinatensystem der Computertomographieeinrichtung gewählt werden können. Hier wird vorgeschlagen, einen Optimierungsalgorithmus einzusetzen, um die relative Höhe, also die Höhenabweichung zur Referenzhöhe, zu ermitteln, für die die höchste Übereinstimmung des wenigstens einen Vergleichsbilds für diese relative Höhe mit dem Zielbereichsbilddatensatz gegeben ist. Die so bestimmte, optimierte relative Höhe entspricht der durch den Zielbereichsbilddatensatz gemessenen Liegenbiegung.

Im bevorzugten Ausführungsbeispiel, in dem im Projektionsraum gearbeitet wird, bedeutet dies konkret, dass für jedes zugeordnete Projektionsbild des Zielbereichsbilddatensatzes mehrere Vergleichsbilder mit unterschiedlichen relativen Höhen des wenigstens einen Markers ermittelt werden, wobei die relative Höhe mit einer höchsten Übereinstimmung des entsprechenden wenigstens einen Vergleichsbilds mit dem zugeordneten wenigstens einen Projektionsbild als Kompensationsinformation bestimmt wird. Mit anderen Worten wird die Höhe des Referenzdatensatzes beziehungsweise der darin beschriebenen Marker und somit des wenigstens einen Vergleichsbilds variiert, beispielsweise während der Vorwärtsprojektion aus dem Referenzdatensatz, und die Liegenbiegung wird abgeschätzt, indem die relative Höhe der Marker aufgefunden wird, die am besten zu den Projektionsdaten des Planungsscans passt.

Konkret kann hierbei die optimierte relative Höhe in einem Optimierungsalgorithmus zur Maximierung der insbesondere durch ein Ähnlichkeits- und/oder Korrelationsmaß beschriebenen Übereinstimmung gewählt werden. Hierbei kann mit besonderem Vorteil vorgesehen sein, dass, insbesondere bei mit Projektionsbildern zu vergleichenden Vergleichsbildern, ein insbesondere zweidimensionales normalisiertes Kreuzkorrelationsmaß verwendet wird. Mithin kann als Kostenfunktion des Optimierungsalgorithmus für die Optimierung der relativen Höhe beispielsweise die zweidimensionale normalisierte Kreuzkorrelation zwischen den Projektionsdaten des Zielbereichsbilddatensatzes und der Vorwärtsprojektion verwendet werden, wobei jedoch auch andere Bildähnlichkeitsmaße beziehungsweise Bildkorrelationsmaße eingesetzt werden können. Die Idee hinter diesem Ansatz ist die Tatsache, dass die Kreuzkorrelation zwischen den Projektionsdaten des Zielbereichsbilddatensatzes und der Vorwärtsprojektion des Referenzdatensatzes ein Maximum erreicht, wenn die Höhe des wenigstens einen Markers in dem Projektionsbild und dem zusätzlichen Vergleichsbild übereinstimmt.

Nachdem diese Annahme dadurch verletzt sein kann, dass die Markerprojektionen durch Merkmale des Objekts, beispielsweise die Patientenanatomie, überlagert sind, kann, wie oben bereits kurz dargelegt, zur Beseitigung dieses Problems und zur allgemeinen Reduzierung des Einflusses der Objektmerkmale, insbesondere der Patientenanatomie, auf die Kreuzkorrelation ein Hochpassfilter auf die Projektionsdaten des Zielbereichsbilddatensatzes sowie die Vergleichsbilder des Referenzdatensatzes angewendet werden, insbesondere ein zweidimensionaler Hochpassfilter. Erst danach wird das Ähnlichkeits- und/oder Korrelationsmaß ermittelt.

Als Optimierungsalgorithmus kann beispielsweise ein Simplex-Algorithmus verwendet werden, wobei auch die Nutzung anderer, im Stand der Technik grundsätzlich bekannter Optimierungsalgorithmen denkbar ist. Der Simplex-Algorithmus ist beispielsweise in einem Artikel von Jeffrey C. Lagarias et al., "Convergence Properties of the Nelder-Mead-Simplex Method in Low Dimensions", SIAM J. OPTIM. Vol. 9, Nr. 1, Seiten 112 bis 147, beschrieben.

In einem anderen Ansatz ist es im Rahmen der vorliegenden Erfindung auch denkbar, den Vergleich sozusagen implizit durchzuführen, indem eine trainierte Funktion, insbesondere also ein Algorithmus der künstlichen Intelligenz, der durch Maschinenlernen trainiert wurde, entsprechend angelernt wird.

Im Allgemeinen bildet eine trainierte Funktion kognitive Funktionen ab, die Menschen mit anderen menschlichen Gehirnen assoziieren. Durch Training basierend auf Trainingsdaten (Maschinenlernen) ist die trainierte Funktion in der Lage, sich an neue Umstände anzupassen und Muster zu detektieren und zu extrapolieren.

Allgemein gesagt können Parameter einer trainierten Funktion durch Training angepasst werden. Insbesondere können überwachtes Lernen, halbüberwachtes Lernen, nicht überwachtes Lernen, Reinforcement Learning und/oder aktives Lernen verwendet werden. Darüber hinaus kann auch Repräsentationslernen (auch als "Feature Learning" bekannt) eingesetzt werden. Die Parameter der trainierten Funktion können insbesondere iterativ durch mehrere Trainingsschritte angepasst werden.

Eine trainierte Funktion kann beispielsweise ein neuronales Netz, eine Support Vector Machine (SVM), einen Entscheidungsbaum und/oder ein Bayes-Netzwerk umfassen und/oder die trainierte Funktion kann auf k-Means-Clustering, Q-Learning, genetischen Algorithmen und/oder Zuordnungsregeln basieren. Insbesondere kann ein neuronales Netzwerk ein tiefes neuronales Netzwerk, ein Convolutional Neural Network (CNN) oder ein tiefes CNN sein. Darüber hinaus kann das neuronale Netzwerk ein Adversarial Network, ein tiefes Adversarial Network und/oder ein Generative Adversarial Network (GAN) sein.

Dabei werden im vorliegenden Anwendungsfall auf tiefem Maschinenlernen (deep learning) basierende Ansätze bevorzugt. Konkret kann beispielsweise vorgesehen sein, dass zur Ermittlung der Kompensationsinformation als Ausgangsdaten eine wenigstens einen Teil des Zielbereichsbilddatensatzes als Eingangsdaten verwendende, auf Grundlage des Referenzdatensatzes trainierte Funktion verwendet wird. Dabei ist es besonders zweckmäßig, wenn als Trainingsdaten für die zu trainierende Funktion Trainingsdatensätze, die den Referenzdatensatz mit auf unterschiedlichen relativen Höhen angeordnetem wenigstens einen Marker umfassen, erzeugt werden.

Mit besonderem Vorteil gilt auch hier, dass die trainierte Funktion bevorzugt auf den Projektionsdaten des Zielbereichsbilddatensatzes arbeitet, mithin diese als Eingangsdaten entgegennimmt. Die trainierte Funktion schätzt, allgemein gesagt, die relative Höhe entsprechend der vorangehend beschriebenen Absätze ab und beschreibt somit die Liegenbiegung. Die trainierte Funktion, insbesondere ein CNN, kann dabei mittels des Referenzdatensatzes bei verschiedenen relativen Höhen als Trainingsdatensätze, insbesondere unter Nutzung daraus abgeleiteter virtueller Projektionsbilder, trainiert werden, so dass die trainierte Funktion gegebene Projektionsdaten als Eingangsdaten in eine relative Höhe übersetzt. Der Vergleich erfolgt dann sozusagen verdeckt innerhalb des neuronalen Netzwerks bzw. allgemein der trainierten Funktion.

Wie bereits erwähnt wurde, kann die Kompensationsinformation insbesondere in einem Verfahren zur Ermittlung einer Lokalisierungsinformation bezüglich des Zielbereichs an einer für einen Vorgang, insbesondere eine Intervention, vorgesehenen, von der Aufnahmeposition unterschiedlichen Vorgangsposition eingesetzt werden, um beispielsweise die Lokalisierungsinformation bzw. bereits den dieser zugrunde liegenden Zielbereichsbilddatensatz entsprechend der ermittelten Liegenbiegung, also der relativen Höhe, zu verschieben. Die Lokalisierungsinformation kann dann beispielsweise zur Ansteuerung von Führungseinrichtungen zur Unterstützung einer den Eingriff durchführenden Person, beispielsweise zur Visualisierung des Zielbereichs bzw. von auf den Zielbereich bezogenen Positionen, und/oder zur Ansteuerung von den Vorgang wenigstens teilweise durchführenden Vorgangseinrichtungen eingesetzt werden, wie beispielsweise hinsichtlich von Lasermarkierungen/Einstellungen für eine Strahlenbehandlung und/oder Anzeigen bezüglich eines minimalinvasiven Instruments und/oder zur Steuerung eines dieses steuernden Roboters.

Bei Vorgängen, bei denen die Vorgangsposition eine weitere Aufnahmeposition zur Aufnahme eines Zusatzbilddatensatzes ist, kann mittels der Erfindung auch eine weitere Kompensationsinformation für die weitere Aufnahmeposition des Zusatzbilddatensatzes erfolgen, so dass die Differenz der so ermittelten Liegenbiegungen den Höhenunterschied von Bildinformationen des Zusatzbilddatensatzes zu dem Zielbereichsbilddatensatz beschreibt.

Neben dem Verfahren betrifft die Erfindung auch eine Computertomographieeinrichtung zum Einsatz bei auf einen Zielbereich eines Objekts, insbesondere eines Patienten, bezogenen Vorgängen, insbesondere Interventionen, aufweisend einen Objekttisch, insbesondere einen Patiententisch, mit einer in zumindest einer Richtung verfahrbaren Liege für das Objekt, wobei an oder in der Liege wenigstens ein in der Röntgenbildgebung sichtbarer Marker vorgesehen ist, und einer Steuereinrichtung, welche aufweist:
- ein Speichermittel, in dem ein dreidimensionaler Referenzdatensatz der Marker bei nicht beladener Liege gespeichert ist,
- eine Aufnahmeeinheit zur Aufnahme eines den Zielbereich des Objekts zeigenden Zielbereichsdatensatzes an einer Aufnahmeposition der Liege,
- eine Kompensationsinformationsermittlungseinheit zur Ermittlung einer Kompensationsinformation zur Kompensation einer Biegung der Liege an der Aufnahmeposition unter dem Gewicht des aufzunehmenden Objekts, wobei die Kompensationsinformationsermittlungseinheit eine Vergleichssubeinheit zur Ermittlung der Kompensationsinformation aus einem Vergleich des Referenzdatensatzes mit dem Zielbereichsbilddatensatz aufweist, und
- eine Kompensationseinrichtung zur Kompensation einer für den Vorgang zu verwendenden, insbesondere anzuzeigenden und/oder anzuzielenden, aus dem Zielbereichsbilddatensatz ermittelten Lokalisierungsinformation bezüglich des Zielbereichs, insbesondere bezüglich einer von der Aufnahmeposition unterschiedlichen Vorgangsposition, aufgrund der Kompensationsinformation.

Sämtliche Ausführungen bezüglich des erfindungsgemäßen Verfahrens lassen sich analog auf die erfindungsgemäße Computertomographieeinrichtung übertragen, so dass auch mit dieser die bereits genannten Vorteile erhalten werden können. Insbesondere können die beschriebenen Funktionseinheiten durch wenigstens einen Prozessor der Steuereinrichtung bereitgestellt werden. Die Steuereinrichtung weist als Funktionseinheit insbesondere auch eine Aufnahmeeinheit auf, die nicht nur allgemein den Aufnahmebetrieb steuern kann, sondern insbesondere den Aufnahmebetrieb für den Zielbereichsbilddatensatz steuert, bei entsprechender Ausgestaltung auch den Aufnahmebetrieb bei der Referenzmessung. Insbesondere kann mithin auch eine Referenzdatensatzermittlungssubeinheit der Kompensationsinformationsermittlungseinheit vorgesehen sein, der die Referenzmessung auswerten kann, gegebenenfalls unter zusätzlicher Berücksichtigung von bereitgestellten Konstruktionsdaten. Allgemein angemerkt kann auch gesagt werden, dass die Steuereinrichtung zur Durchführung eines erfindungsgemäßen Verfahrens ausgebildet ist und zudem Komponenten zur Anwendung der Kompensationsinformation aufweist.

Ein erfindungsgemäßes Computerprogramm ist beispielsweise direkt in ein Speichermittel einer Steuereinrichtung einer Computertomographieeinrichtung ladbar und weist Programmmittel auf, um die Schritte eines erfindungsgemäßen Verfahrens durchzuführen, wenn das Computerprogramm auf der Steuereinrichtung der Computertomographieeinrichtung ausgeführt wird. Das Computerprogramm kann auf einem erfindungsgemäßen elektronisch lesbaren Datenträger gespeichert sein, welcher mithin Steuerinformationen umfasst, die wenigstens ein erfindungsgemäßes Computerprogramm umfassen und derart ausgestaltet sind, dass bei Verwendung des Datenträgers in einer Steuereinrichtung einer Computertomographieeinrichtung diese ausgebildet wird, ein erfindungsgemäßes Verfahren durchzuführen. Bei dem elektronisch lesbaren Datenträger kann es sich beispielsweise um einen nichttransienten Datenträger, insbesondere eine CD-ROM, handeln.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: einen Ablaufplan eines Ausführungsbeispiels des erfindungsgemäßen Verfahrens,
- Fig. 2: eine schematische Skizze einer erfindungsgemäßen Computertomographieeinrichtung bei einer ersten Liegenposition,
- Fig. 3: eine schematische Skizze der Computertomographieeinrichtung bei einer zweiten Liegenposition,
- Fig. 4: einen Querschnitt durch einen Patiententisch der Computertomographieeinrichtung,
- Fig. 5: einen Längsschnitt durch eine Liege des Patiententischs der Fig. 4,
- Fig. 6: Schnittbilder eines Referenzdatensatzes und eines Zielbereichsbilddatensatzes, und
- Fig. 7: schematisch die funktionale Struktur einer Steuereinrichtung der Computertomographieeinrichtung.

Im Folgenden sollen Ausführungsbeispiele der vorliegenden Erfindung für eine Anwendung, hier eine Intervention als Vorgang, im medizinischen Bereich genauer dargelegt werden. Das bedeutet, das Objekt entspricht dort einem Patienten, wobei in einem Zielbereich des Patienten als Intervention beispielsweise eine Strahlentherapie und/oder ein minimalinvasiver Eingriff mit einem medizinischen Instrument (medizinischen Eingriffsinstrument) erfolgen soll. Bei dem Objekttisch handelt es sich somit im Folgenden beispielhaft um einen Patiententisch. Neben den hier beschriebenen Anwendungsfällen im medizinischen Bereich ist auch ein Vorgang. Insbesondere eine Intervention, an sonstigen Objekten, beispielsweise im Hinblick auf eine Materialprüfung und/oder Materialbehandlung, grundsätzlich denkbar.

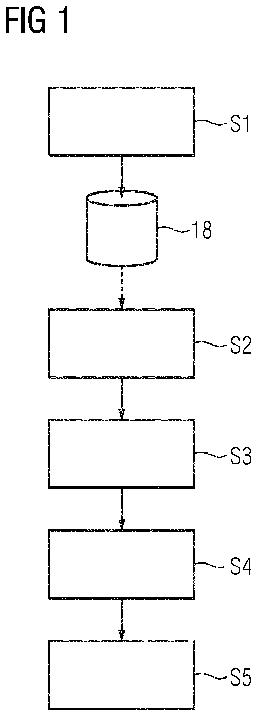

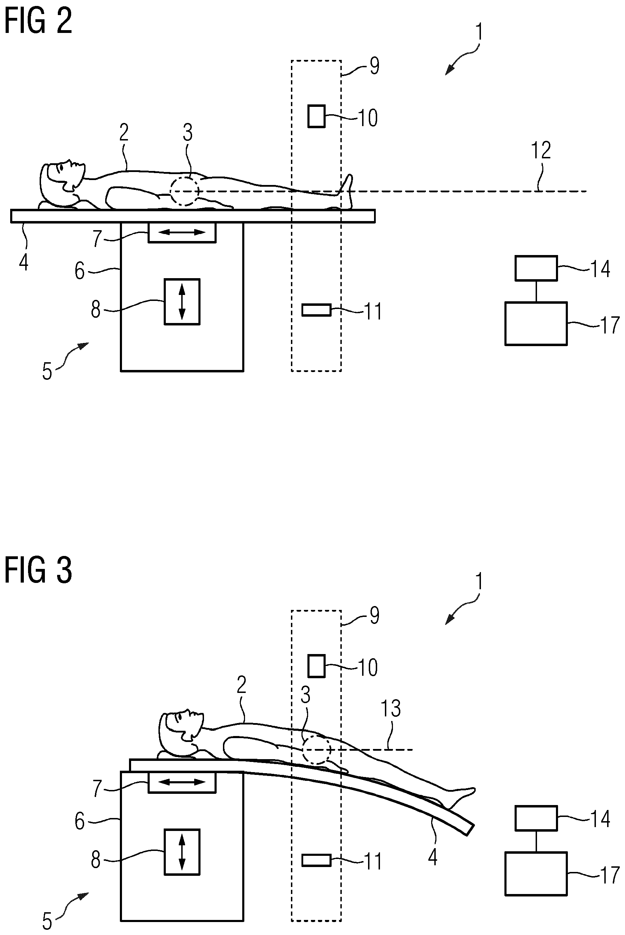

Fig. 1 zeigt einen beispielhaften Ablaufplan eines Ausführungsbeispiels des erfindungsgemäßen Verfahrens sowie gegebenenfalls umgebender Schritte. Bevor diese im Einzelnen diskutiert werden, soll jedoch zunächst im Hinblick auf Fig. 2 und Fig. 3, die jeweils stark schematisch vereinfachte Ansichten einer erfindungsgemäßen Computertomographieeinrichtung 1 zeigen, das der Erfindung zugrunde liegende Problem erläutert werden.

Vorliegend soll, wie bereits erwähnt, an einem (nicht zur Computertomographieeinrichtung 1 gehörenden) Patienten 2 eine Intervention in einem Zielbereich 3, beispielsweise an einem Tumor oder einer sonstigen Läsion, vorgenommen werden. Der Patient 2 wird dazu in einer bestimmten Position und Orientierung auf einer Liege 4 eines Patiententisches 5 angeordnet, wobei auf der Liege 4 auch noch zusätzlich eine hier nicht näher gezeigte Zusatzplatte, beispielsweise ein sogenanntes Overlay, vorgesehen sein kann. Der Patiententisch 5 umfasst neben der Liege 4 eine Trägerstruktur 6, beispielsweise wenigstens einen Tischfuß, auf der die Liege 4 mittels entsprechender erster Einstellmittel 7 in Längsrichtung der Liege 4 verfahrbar ist, mittels entsprechender zweiter Einstellmittel 8 in Höhenrichtung einstellbar ist. Die Längsrichtung ist dabei parallel zu einer Rotationsachse einer in einer hier nur gestrichelt angedeuteten Gantry vorgesehenen Aufnahmeanordnung, die vorliegend neben der Röntgenquelle 10 auch einen Röntgendetektor 11 umfasst. Die Intervention an dem Zielbereich 3 kann hierbei nicht bei innerhalb der Gantry im Sichtfeld der Computertomographieeinrichtung 1 befindlichem Zielbereich 3 durchgeführt werden, da der Zugang zu eingeschränkt ist. Vielmehr soll als Vorgangsposition eine in Fig. 2 dargestellte Interventionsposition der Liege 4 genutzt werden, in der vorliegend der Zielbereich 3 oberhalb der Trägerstruktur 6 positioniert ist.

Zur Planung der Intervention ist jedoch auch die Aufnahme eines Zielbereichsbilddatensatzes in einem Planungsscan durchzuführen, wozu die Liege 4 in eine in Fig. 3 schematisch angedeutete Aufnahmeposition zu verfahren ist, in der sich der Zielbereich 3 im Sichtfeld der Aufnahmeanordnung (Röntgenquelle 10, Röntgendetektor 11) der Computertomographieeinrichtung 1, also innerhalb der Gantry 9, genauer einer dortigen Objektöffnung, befindet. Hierbei ragt die Liege 4 freitragend in die Objektöffnung der Gantry 9 ein, so dass es aufgrund der Schwerkraft, mithin des Gewichts des Patienten 2, zu einer leichten, in Fig. 3 aus Darstellungszwecken übertrieben dargestellten Biegung der Liege 4 kommt. Das bedeutet, während sich in der in Fig. 2 schematisch dargestellten Interventionsposition der Zielbereich 3 auf einer ersten Höhe 12 befindet, befindet er sich aufgrund der Liegenbiegung in der in Fig. 3 schematisch dargestellten Aufnahmeposition auf einer zweiten, niedrigeren Höhe 13, so dass mithin eine Höhenabweichung zwischen dem Zielbereichsbilddatensatz und der realen Position des Zielbereichs 3 in der Interventionsposition besteht.

Wird nun durch Auswerten des Zielbereichsbilddatensatzes ohne jegliche Kompensation dieser Höhenabweichung aufgrund der Liegenbiegung eine Lokalisierungsinformation bezüglich des Zielbereichs 3 ermittelt, liegen die darin enthaltenen Positionen leicht zu niedrig. Dies kann zu Ungenauigkeiten bei der Intervention führen, beispielsweise bei der Visualisierung der Lokalisierungsinformation mittels einer entsprechenden, insbesondere laserbasierten Führungseinrichtung 14.

Daher ist in den hier dargestellten Ausführungsbeispielen vorgesehen, die Höhenabweichung durch die Liegenbiegung als eine Kompensationsinformation zu ermitteln und eine entsprechende Verschiebung des Zielbereichsbilddatensatzes bzw. der daraus abgeleiteten Lokalisierungsinformation als Kompensation vorzusehen.

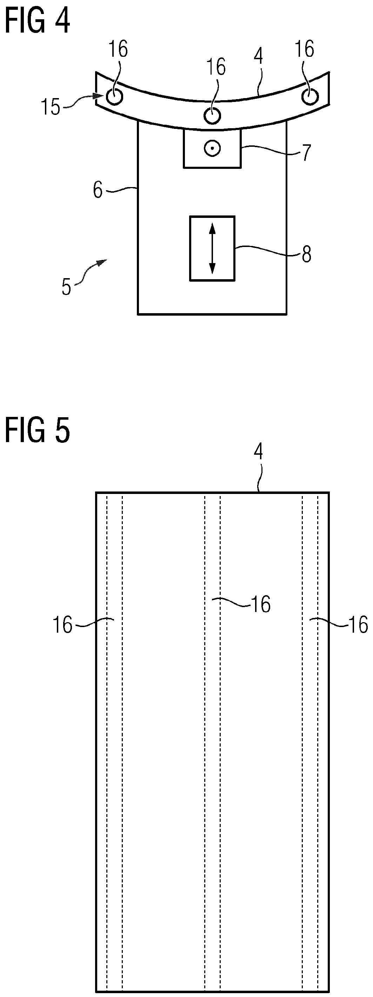

Hierzu ist zunächst in der erfindungsgemäßen Computertomographieeinrichtung 1 eine spezielle Ausgestaltung der Liege 4 des Patiententisches 5 vorgesehen, wie die Figuren 4 und 5 zeigen. In oder an der Liege 4, vorliegend in die Liege 4 integriert, sind als wenigstens ein Marker 15 drei Markerelemente 16, die in der Röntgenbildgebung sichtbar sind, integriert.

Bei den Markerelementen 16 handelt es sich im vorliegenden Ausführungsbeispiel um längliche, drahtartige Kunststoffzylinder mit einem HU-Wert beispielsweise im Bereich von -600 bis 100, so dass keine zu starke, Artefakte zur Folge habende Absorption der Röntgenstrahlung gegeben ist. Die länglichen Markerelemente 16 erstrecken sich dabei mit ihrer Längsrichtung parallel zur Längsrichtung der Liege 4, die ja der Verfahrrichtung mittels der Einstellmittel 7 zwischen der Aufnahmeposition und der Interventionsposition entspricht. Somit sehen die Markerelemente 16 unabhängig von der tatsächlichen Aufnahmeposition in der Röntgenbildgebung immer gleich aus. Sie erstrecken sich im Ausführungsbeispiel über die gesamte Länge der Liege 4, können jedoch auch nur den Bereich bei möglichen Aufnahmepositionen im Sichtfeld befindlicher Liegenabschnitte der Liege 4 umfassen. Die Markerelemente 16 können beispielsweise in ein Liegenmaterial der Liege 4 eingegossen sein oder aber auch in einer verschlossenen Nut der Liege 4 angeordnet sein.

Selbstverständlich sind auch andere konkrete Ausgestaltungen der Markerelemente 16 denkbar, beispielsweise andere längliche, parallel zur Verfahrrichtung zwischen der Interventionsposition und der Aufnahmeposition ausgerichtete Formen und/oder andere Anzahlen, nachdem nur beispielhaft in diesem Ausführungsbeispiel drei drahtartige Markerelemente 16 gezeigt sind, von denen eines mittig, eines rechts und das andere links angeordnet sind.

Es sei noch angemerkt, dass der Betrieb der Computertomographieeinrichtung 1 durch eine Steuereinrichtung 17 gesteuert wird, die auch zur Durchführung des im Hinblick auf Fig. 1 nun näher beschriebenen Verfahrens zur Ermittlung der Kompensationsinformation sowie der weiteren Schritte ausgebildet ist. Die Steuereinrichtung 17 kann auch eine Steuereinheit des Patiententisches 5 umfassen.

In einem für jeden Patiententisch 5 und dessen Anordnung in der Computertomographieeinrichtung 1 nur einmal durchzuführenden Schritt S1 gemäß Fig. 1 wird dabei zunächst ein Referenzdatensatz 18 aufgrund einer Referenzmessung ermittelt. Die mit der Aufnahmeanordnung in der Gantry 9 der Computertomographieeinrichtung 1 durchgeführte Referenzmessung ist dabei eine übliche Computertomographiemessung, nur dass kein Objekt auf der Liege 4 befindlich ist und die Höheneinstellung mittels der Einstellmittel 8 so gewählt wurde, dass sich die Markerelemente 16 als wenigstens ein Marker 15 im Zentrum des Sichtfeldes der Computertomographieeinrichtung 1 befinden, mithin die Markerelemente 16 in jedem Projektionsbild der Referenzmessung zu sehen sind. Dies ermöglicht eine vollständige Rekonstruktion eines dreidimensionalen Rekonstruktionsbildes aus den Projektionsbildern der Referenzmessung, welches zunächst sowohl die Markerelemente 16 als auch den restlichen Tisch 4 zeigt.

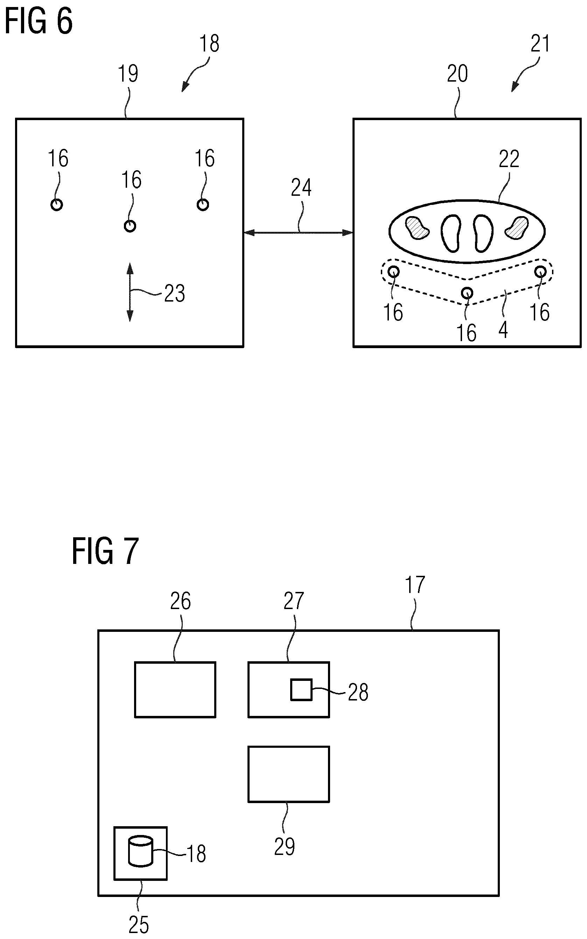

Gegebenenfalls unter Hilfe von Konstruktionsdaten des Patiententisches 5, insbesondere der Liege 4, werden die Markerelemente 16 nun segmentiert. Alle Bereiche außerhalb der segmentierten Markerelemente werden zum Erhalt eines Referenzdatensatzes 18 dann auf den HU-Wert von Luft, also -1000, gesetzt. Der Referenzdatensatz 18 enthält in diesem Ausführungsbeispiel also lediglich die Markerelemente 16 als der wenigstens eine Marker 15. Diesbezüglich zeigt Fig. 6 auf der linken Bildseite beispielhaft ein Schnittbild 19 aus dem Referenzdatensatz 18, und zwar senkrecht zur Längsrichtung/Verfahrrichtung der Liege 4, wo die länglichen Markerelemente 16 mithin in ihrem Querschnitt in der auch Fig. 4 zu entnehmenden Anordnung zu sehen sind.

Der Referenzdatensatz 18 wird in einem Speichermittel der Steuereinrichtung 17 gespeichert, gemeinsam mit der Referenzhöheneinstellung des Patiententisches 5, die hierfür gegeben war.

Steht nun eine Intervention in einem Zielbereich 3 eines Patienten 2 bevor, kann jedes Mal das nun beschriebene Vorgehen auf der Basis desselben Referenzdatensatzes 18 eingesetzt werden, insbesondere auch unabhängig davon, ob ein Overlay verwendet wird oder nicht. Hierfür wird der Referenzdatensatz 18 aus dem Speichermittel bereitgestellt und gegebenenfalls bei einer Abweichung der Höheneinstellung in der Aufnahmeposition von der Referenzhöheneinstellung gemäß dieser Abweichung verschoben, so dass sich die Marker 15, hier Markerelemente 16, im Referenzdatensatz 18 auf der Referenzhöhe befinden, auf der sie angeordnet wären, wenn sich kein Objekt auf der Liege 4 befinden würde.

Der Schritt S2 zeigt nun zunächst an, dass der Zielbereichsbilddatensatz in der Aufnahmeposition aufgenommen wird. Zu diesem Zielbereichsbilddatensatz wird unter Verwendung desselben nun in einem Schritt S3 eine Kompensationsinformation, die die Liegenbiegung beschreibt, ermittelt, indem letztlich ein Vergleich des Referenzdatensatzes 18 mit dem Zielbereichsbilddatensatz für verschiedene relative Höhen, hier selbstverständlich Absenkungen, im Vergleich zur Referenzhöhe durchgeführt werden und die relative Höhe, für die die größte Übereinstimmung gegeben ist, als Kompensationsinformation bestimmt wird, die die Liegenbiegung und damit auch die Höhenabweichung bezüglich des Zielbereichs 3 beschreibt.

Dieses grundlegende Prinzip wird im Hinblick auf Fig. 6 näher erläutert, wo auf der rechten Seite neben dem Schnittbild 19 des Referenzdatensatzes 18 auch ein entsprechendes Schnittbild 20 des Zielbereichsbilddatensatzes 21 gezeigt ist. Der Zielbereichsbilddatensatz 21 zeigt neben den erneut sichtbaren Markerelementen 16 schwach auch die Liege 4 sowie Anatomie 22 des Patienten 2. Die relative Höhe wird nun gemäß dem Pfeil 23 variiert, bis eine optimale Übereinstimmung bei dem durch den Pfeil 24 symbolisierten Vergleich gegeben ist, wobei der Vergleich vorliegend nicht auf Schnittbildern 19, 20 basiert, sondern bevorzugt im Projektionsraum, also bezüglich der Projektionsdaten, durchgeführt wird. Hinsichtlich der Variation der relativen Höhe wird im Allgemeinen ein Optimierungsalgorithmus eingesetzt, um die optimale Übereinstimmung anzeigende relative Höhe (optimierte relative Höhe) als Kompensationsinformation zu bestimmen. Dies alles sei nun anhand eines konkreten Ausführungsbeispiels näher erläutert.

Um die Liegenbiegung für den gegebenen Planungsscan, also den Zielbereichsbilddatensatz 21, zu ermitteln, wird zunächst eine simulierte Vorwärtsprojektion des Referenzdatensatzes 18, genauer der darin enthaltenen Markerelemente 16, unter Verwendung der Aufnahmegeometrien der Projektionsbilder des tatsächlichen Planungsscans durchgeführt. Im Ergebnis erhält man für jede "auszutestende" relative Höhe, insbesondere Absenkung zur Referenzhöhe, einen Satz von vorwärtsprojizierten Vergleichsbildern, von denen jedes einem Projektionsbild gleicher Aufnahmegeometrie in dem Zielbereichsbilddatensatz 21 zugeordnet ist. Die Gesamtheit der Projektionsbilder des Zielbereichsbilddatensatzes 21 können dabei als Projektionsdatensatz, die Gesamtheit der Vergleichsbilder als Vergleichsdatensatz verstanden werden. Die Projektionsdaten werden nun mit diesen Vergleichsdaten verglichen, wobei die Höhe der Marker 15 in dem Referenzdatensatz 18 während der Vorwärtsprojektion variiert wird und die Tischbiegung durch Auffinden der relativen Höhe der Markerelemente 16 des Referenzdatensatzes 18 abgeschätzt wird, die am besten zu den Projektionsdaten des Zielbereichsbilddatensatzes 21 passt. Hier wird ein Optimierungsalgorithmus eingesetzt, dessen Kostenfunktion ein Ähnlichkeits- und/oder Korrelationsmaß umfasst und somit maximiert wird. In der vorliegenden konkreten Ausführungsform kann dabei die zweidimensionale normalisierte Kreuzkorrelation zwischen den Projektionsdaten des Zielbereichsbilddatensatzes 21 und den Vergleichsdaten der Vorwärtsprojektion des Referenzdatensatzes 18 verwendet werden, wobei auch andere Maße grundsätzlich denkbar sind.

Die Annahme, dass die Kreuzkorrelation (oder allgemeine Übereinstimmung) zwischen den Projektionsbildern und den Vergleichsbildern ein Maximum erreicht, wenn die Höhe der Markerelemente 16 in den zugeordneten Projektions- und Vergleichsbildern übereinstimmt, kann dadurch die Frage gestellt sein, dass die Markerprojektionen in den Projektionsbildern durch die Patientenanatomie 22 überlagert sein können. Um dieses Problem zu lösen und den Einfluss der Patientenanatomie 22 auf die Kreuzkorrelation grundsätzlich zu reduzieren, wird auf die Projektionsbilder und die Vergleichsbilder, konkret den Vergleichsdatensatz und den Projektionsdatensatz, vor dem Vergleich ein zweidimensionaler Hochpassfilter angewendet. Dabei kann als Hochpassfilter beispielsweise zunächst ein zweidimensionaler Gaußscher Tiefpassfilter angewendet werden und das Ergebnis von den Originaldaten abgezogen werden. Selbstverständlich können auch andere Hochpassfilter eingesetzt werden.

Zur Ermittlung der optimierten relativen Höhe kann dabei jeder numerische Optimierungsalgorithmus eingesetzt werden, wobei vorliegend der Simplex-Algorithmus eingesetzt wird.

Es sei schließlich bezüglich des Schrittes S3, Ermittlung der Kompensationsinformation, auch darauf hingewiesen, dass auch der Einsatz einer trainierten Funktion, die implizit den Vergleich realisiert, denkbar ist. Beispielsweise kann ein CNN aufgrund des Referenzdatensatzes 18 mit unterschiedlichen Höhen, woraus Trainingsdatensätze abgeleitet werden, trainiert werden, wobei in der Anwendung dem CNN die Projektionsdaten übergeben werden und als Ausgabe die optimierte relative Höhe als Kompensationsinformation erhalten wird.

Zurückkehrend zu Fig. 1 kann in einem Schritt S4 eine Anwendung der Kompensationsinformation erfolgen, nachdem diese die optimierte relative Höhe beschreibt, die auch die im Hinblick auf die Figuren 2 und 3 beschriebene Höhenabweichung des Zielbereichs 3 ist. Mithin kann die Kompensationsinformation in dem Sinne zur Kompensation einer Lokalisierungsinformation bezüglich des Zielbereichs eingesetzt werden, dass beispielsweise bereits vor Ermittlung der Lokalisierungsinformation eine korrigierende Höhenverschiebung des Zielbereichsbilddatensatzes 21 um die optimierte relative Höhe erfolgt oder aber eine Verschiebung der ermittelten Lokalisierungsinformation um die als Kompensationsinformation ermittelte optimierte relative Höhe vorgenommen wird.

In einem Schritt S5 kann die so ermittelte, bezüglich der Höhe aufgrund der Liegenbiegung unter dem Patienten 2 kompensierte Lokalisierungsinformation insbesondere zur Ansteuerung der Führungseinrichtung 14, die an der Interventionsposition als Vorgangsposition wirkt, eingesetzt werden. Beispielsweise können Lasermarkierungen zum Einführen eines minimalinvasiven Instruments, beispielsweise einer Nadel für eine Biopsie, und/oder für eine Strahlentherapie korrekt mittels der Führungseinrichtung visualisiert werden und/oder es kann auch eine Steuerung einer Vorgangseinrichtung, insbesondere einer Bestrahlungseinrichtung und/oder eines das minimalinvasive Instrument steuernden Roboters, aufgrund der korrigierten Lokalisierungsinformation korrekt und zur qualitativ hochwertigen Durchführung der Intervention erfolgen.

Fig. 7 zeigt rein schematisch die funktionale Struktur der Steuereinrichtung 17. Diese weist zunächst, wie bereits erwähnt, ein Speichermittel 25 auf, in dem beispielsweise der Referenzdatensatz 18 abgespeichert werden kann, insbesondere gemeinsam mit der Referenzhöheneinstellung. In einer Aufnahmeeinheit 26 der Steuereinrichtung 17 wird der Aufnahmebetrieb der Computertomographieeinrichtung 1 gesteuert, insbesondere auch die Referenzmessung des Schrittes S1 und die Aufnahme des Zielbereichsbilddatensatzes 21 gemäß Schritt S2.

Die Steuereinrichtung 17 weist ferner eine Kompensationsinformationsermittlungseinheit 27 zur Ermittlung der Kompensationsinformation gemäß Schritt S3 auf, die als Untereinheiten eine Vergleichssubeinheit 28 und/oder eine Referenzdatensatzermittlungssubeinheit (hier nicht gezeigt) umfassen kann. In einer Kompensationseinheit 29 kann dann die Kompensation gemäß dem Schritt S4 erfolgen.

Die Steuereinrichtung 17 kann selbstverständlich auch weitere Funktionseinheiten aufweisen, beispielsweise eine Lokalisierungsinformationsermittlungseinheit zur Ermittlung der Lokalisierungsinformation und diverse Ansteuereinheiten, beispielsweise auch zur Durchführung des Schrittes S5.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Ermittlung einer Kompensationsinformation zur Kompensation einer Biegung einer Liege (4) eines Objekttischs, insbesondere Patiententisches (5), einer Computertomographieeinrichtung (1) unter dem Gewicht eines aufzunehmenden Objekts, insbesondere eines Patienten (2), wobei an einer Aufnahmeposition der Liege (4) ein Zielbereichsbilddatensatz (21) eines Zielbereichs (3) des Untersuchungsobjekts mit der Computertomographieeinrichtung (1) aufgenommen wird, wobei an oder in der Liege (4) wenigstens ein in der Röntgenbildgebung sichtbarer Marker (15) vorgesehen ist und ein dreidimensionaler Referenzdatensatz (18) der Marker (15) bei nicht beladener Liege (4) bereitgestellt wird, wobei die Kompensationsinformation aus einem Vergleich des Referenzdatensatzes (18) mit dem Zielbereichsbilddatensatz (21) ermittelt wird, **dadurch gekennzeichnet, dass** zum Vergleich des Referenzdatensatzes (18) mit dem Zielbereichsbilddatensatz (21) wenigstens ein Vergleichsbild mit einer Aufnahmegeometrie eines zugeordneten Projektionsbilds des Zielbereichsbilddatensatzes (21) durch Vorwärtsprojektion aus dem Referenzdatensatz (18) ermittelt wird und mit dem zugeordneten Projektionsbild im Projektionsraum verglichen wird.

2. Verfahren nach Anspruch 1, **dadurch gekennzeichnet, dass** der Referenzdatensatz (18) in einer, insbesondere bei einer erstmaligen Inbetriebnahme der Computertomographieeinrichtung (1) mit aktuellen Liegeneigenschaften durchgeführten, Referenzmessung bei nicht beladener Liege (4) ermittelt wird und/oder aus Konstruktionsdaten des Objekttisches bereitgestellt wird.

3. Verfahren nach Anspruch 2, **dadurch gekennzeichnet, dass** für die Referenzmessung der in ihrer Höhe verstellbaren Liege (4) eine Referenzhöheneinstellung gewählt wird, so dass der wenigstens eine Marker (15) in jedem Projektionsbild der Referenzmessung erfasst wird, wobei der Referenzdatensatz (18) für die Ermittlung der Kompensationsinformation bei einer Abweichung der Höheneinstellung von der Referenzhöheneinstellung um diese Abweichung in Höhenrichtung verschoben wird.

4. Verfahren nach einem der vorangehenden Ansprüche, **dadurch gekennzeichnet, dass** als Marker (15) wenigstens ein längliches, insbesondere drahtartiges, sich in einer, insbesondere zu einer Rotationsachse einer Röntgenquelle (10) der Computertomographieeinrichtung (1) parallelen und/oder einer Längsrichtung der Liege (4) entsprechenden, Verfahrrichtung der Liege (4) erstreckendes Markerelement (16) verwendet wird.

5. Verfahren nach Anspruch 4, **dadurch gekennzeichnet, dass** sich das Markerelement (16) zumindest im Wesentlichen über die gesamte Länge der Liege (4) erstreckt und/oder das Markerelement (16) in die Liege (4) integriert ist und/oder wenigstens drei parallele Markerelemente (16) verwendet werden, insbesondere eines mittig und zwei, bevorzugt symmetrisch, benachbart hierzu, bevorzugt in Randbereichen der Liege (4), und/oder auf unterschiedlichen Höhen innerhalb der Liege (4).

6. Verfahren nach einem der vorangehenden Ansprüche, **dadurch gekennzeichnet, dass** der Referenzdatensatz (18) nur den wenigstens einen Marker (15) zeigend ermittelt wird.

7. Verfahren nach einem der vorangehenden Ansprüche, **dadurch gekennzeichnet, dass** zum Vergleich des Referenzdatensatzes (18) mit dem Zielbereichsbilddatensatz (21) für mehrere, bevorzugt alle, wenigstens einen des wenigstens einen Markers (15) zeigenden Projektionsbilder des Zielbereichsbilddatensatzes (21) wenigstens ein Vergleichsbild mit einer Aufnahmegeometrie des zugeordneten Projektionsbilds des Zielbereichsbilddatensatzes (21) durch Vorwärtsprojektion aus dem Referenzdatensatz (18) ermittelt wird und mit dem zugeordneten Projektionsbild im Projektionsraum verglichen wird, wobei für jedes zugeordnete Projektionsbild des Zielbereichsbilddatensatzes mehrere Vergleichsbilder mit unterschiedlichen relativen Höhen des wenigstens einen Markers ermittelt werden, wobei die relative Höhe mit einer höchsten Übereinstimmung des entsprechenden wenigstens einen Vergleichsbilds mit dem zugeordneten wenigstens einen Projektionsbild als Kompensationsinformation bestimmt wird.

8. Verfahren nach einem der vorangehenden Ansprüche, **dadurch gekennzeichnet, dass** die Vergleichsbilder und die Projektionsbilder vor dem Vergleich unter Verwendung eines gleichen Filters hochpassgefiltert werden.

9. Verfahren nach einem der vorangehenden Ansprüche, **dadurch gekennzeichnet, dass** aus dem Referenzdatensatz (18) unter Berücksichtigung der Aufnahmegeometrie für den Zielbereichsbilddatensatz (21) Vergleichsbilder für unterschiedliche relative Höhen des wenigstens einen Markers (15) ermittelt werden, wobei eine optimierte relative Höhe mit einer höchsten Übereinstimmung des wenigstens einen entsprechenden Vergleichsbilds mit wenigstens einem entsprechenden Zielbereichsbild des Zielbereichsbilddatensatzes (21) als Kompensationsinformation bestimmt wird.

10. Verfahren nach Anspruch 9, **dadurch gekennzeichnet, dass** die optimierte Höhe in einem Optimierungsalgorithmus zur Maximierung der insbesondere durch ein Ähnlichkeits- und/oder Korrelationsmaß beschriebenen Übereinstimmung gewählt wird.

11. Verfahren nach einem der vorangehenden Ansprüche, **dadurch gekennzeichnet, dass** zur Ermittlung der Kompensationsinformation als Ausgangsdaten eine wenigstens einen Teil des Zielbereichsbilddatensatzes (21) als Eingangsdaten verwendende, auf Grundlage des Referenzdatensatzes (18) trainierte Funktion verwendet wird.

12. Verfahren nach Anspruch 11, **dadurch gekennzeichnet, dass** als Trainingsdaten für die zu trainierende Funktion Trainingsdatensatze, die den Referenzdatensatz (18) mit auf unterschiedlichen relativen Höhen angeordnetem wenigstens einen Marker (15) umfassen, erzeugt werden.

13. Computertomographieeinrichtung (1) zum Einsatz bei auf einen Zielbereich (3) eines Objekts, insbesondere eines Patienten (2), bezogenen Vorgängen, insbesondere Interventionen, aufweisend einen Objekttisch, insbesondere Patiententisch (5), mit einer in zumindest einer Richtung verfahrbaren Liege (4) für das Objekt, wobei an oder in der Liege (4) wenigstens ein in der Röntgenbildgebung sichtbarer Marker (15) vorgesehen ist, und einer Steuereinrichtung (17), welche aufweist:
- ein Speichermittel (25), in dem ein dreidimensionaler Referenzdatensatz (18) der Marker (15) bei nicht beladener Liege (4) gespeichert ist,
- eine Aufnahmeeinheit zur Aufnahme eines den Zielbereich (3) des Objekts zeigenden Zielbereichsdatensatzes (21) an einer Aufnahmeposition der Liege (4),
- eine Kompensationsinformationsermittlungseinheit (27) zur Ermittlung einer Kompensationsinformation zur Kompensation einer Biegung der Liege (4) an der Aufnahmeposition unter dem Gewicht des aufzunehmenden Objekts, wobei die Kompensationsinformationsermittlungseinheit (27) eine Vergleichssubeinheit (28) zur Ermittlung der Kompensationsinformation aus einem Vergleich des Referenzdatensatzes (18) mit dem Zielbereichsbilddatensatz (21) aufweist, und
- eine Kompensationseinrichtung (29) zur Kompensation einer für den Vorgang zu verwendenden, aus dem Zielbereichsbilddatensatz (21) ermittelten Lokalisierungsinformation bezüglich des Zielbereichs (3), insbesondere bezüglich einer von der Aufnahmeposition unterschiedlichen Vorgangsposition, aufgrund der Kompensationsinformation,
- **dadurch gekennzeichnet, dass** die Computertomographieeinrichtung (1) dazu eingerichtet ist, zum Vergleich des Referenzdatensatzes (18) mit dem Zielbereichsbilddatensatz (21) wenigstens ein Vergleichsbild mit einer Aufnahmegeometrie eines zugeordneten Projektionsbilds des Zielbereichsbilddatensatzes (21) durch Vorwärtsprojektion aus dem Referenzdatensatz (18) zu ermitteln und mit dem zugeordneten Projektionsbild im Projektionsraum zu vergleichen.

14. Computerprogramm, welches die Schritte eines Verfahrens nach einem der Ansprüche 1 bis 12 durchführt, wenn es auf einer Steuereinrichtung (17) einer Computertomographieeinrichtung (1) ausgeführt wird.

15. Elektronisch lesbarer Datenträger, auf dem ein Computerprogramm nach Anspruch 14 gespeichert ist.

## Claims

1. Method for establishing compensation information to compensate for a bending of a couch (4) of an object table, in particular a patient table (5), of a computed tomography facility (1) under the weight of an object to be recorded, in particular of a patient (2), wherein a target region image dataset (21) of a target region (3) of the examination object is recorded with the computed tomography facility (1) at a recording position of the couch (4), wherein at least one marker (15) visible in x-ray imaging is provided at or in the couch (4) and a three-dimensional reference dataset (18) of the marker (15) for a non-loaded couch (4) is provided, wherein the compensation information is established from a comparison between the reference dataset (18) and the target region image dataset (21),
**characterised in that**
for comparing the reference dataset (18) with the target region image dataset (21), at least one comparison image with a recording geometry of an assigned projection image of the target region image dataset (21) is established by forward projection from the reference dataset (18) and is compared with the assigned projection image in the projection space.

2. Method according to claim 1, **characterised in that** the reference dataset (18) is established in a reference measurement for a non-loaded couch (4), in particular carried out during an initial commissioning of the computed tomography facility (1) with current couch characteristics and/or is provided from construction data of the object table.

3. Method according to claim 2, **characterised in that** for the reference measurement of the couch (4) adjustable in its height, a reference height setting is selected, so that the at least one marker (15) is acquired in each projection image of the reference measurement, wherein the reference dataset (18), for establishing the compensation information for a deviation of the height setting from the reference height setting, is shifted by this deviation in the height direction.

4. Method according to one of the preceding claims, **characterised in that** at least one elongated, in particular wire-like marker element (16), extending in particular in a direction of movement of the couch (4) in parallel to an axis of rotation of an x-ray source (10) of the computed tomography facility (1) and/or corresponding to a longitudinal direction of the couch (4) is used as the marker (15).

5. Method according to claim 4, **characterised in that** the marker element (16) extends at least essentially over the entire length of the couch (4) and/or the marker element (16) is integrated into the couch (4) and/or at least three parallel marker elements (16) are used, in particular one in the middle and two, preferably symmetrically, adjacent hereto, preferably in edge areas of the couch (4), and/or at different heights within the couch (4).

6. Method according to one of the preceding claims, **characterised in that** the reference dataset (18) is established showing only the at least one marker (15).

7. Method according to one of the preceding claims, **characterised in that**, for comparison of the reference dataset (18) with the target region image dataset (21), at least one comparison image with a recording geometry of an assigned projection image of the target region image dataset (21) is established by forward projection from the reference dataset (18) and is compared with the assigned projection image in the projection space, for a number of, preferably all, projection images of the target region image dataset (21) showing at least one of the at least one marker (15), wherein for each assigned projection image of the target region image dataset, a number of comparison images is established with different relative heights of the at least one marker, wherein the relative height with a highest match between the at least one corresponding comparison image and the at least one assigned projection image is determined as compensation information.

8. Method according to one of the preceding claims, **characterised in that** the comparison images and the projection images are highpass filtered before the comparison using an identical filter.

9. Method according to one of the preceding claims, **characterised in that** comparison images for different relative heights of the at least one marker (15) are established from the reference dataset (18), taking into account the recording geometry for the target region image dataset (21), wherein an optimized relative height with a highest match between the at least one corresponding comparison image and the least one corresponding target region image of the target region image dataset (21) is determined as compensation information.

10. Method according to claim 9, **characterised in that** the optimized height is selected in an optimization algorithm for maximization of the match described in particular by a measure of similarity and/or correlation.

11. Method according to one of the preceding claims, **characterised in that**, to establish the compensation information a trained function using at least one part of the target region image dataset (21) as input data, trained on the basis of the reference dataset (18) is used as output data.

12. Method according to claim 11, **characterised in that**, as training data for the function to be trained, training datasets, which comprise the reference dataset (18) with at least one marker (15) arranged at different relative heights, are created.

13. Computed tomography facility (1) for use in processes related to a target region (3) of an object, in particular of a patient (2), in particular interventions, having an object table, in particular patient table (5), with a couch (4) for the object able to be moved in at least one direction, wherein at least one marker (15) visible in the x-ray imaging is provided on or in the couch (4), and a control facility (17), which has:
- a storage means (25), in which a three-dimensional reference dataset (18) of the marker (15) for a non-loaded couch (4) is stored,
- a recording unit for recording a target region image dataset (21) showing the target region (3) of the object at a recording position of the couch (4),
- a compensation information establishment unit (27) for establishing compensation information to compensate for a bending of the couch (4) at the recording position under the weight of the object to be recorded, wherein the compensation information establishment unit (27) has a comparison subunit (28) for establishing the compensation information from a comparison of the reference dataset (18) with the target region image dataset (21), and
- a compensation facility (29) to compensate for localization information relating to the target region (3), in particular relating to a process position different from the recording position, to be used for the process, established from the target region image dataset (21) on the basis of the compensation information,
- **characterised in that** for comparing the reference dataset (18) with the target region image dataset (21), the computed tomography facility (1) is configured to establish at least one comparison image with a recording geometry of an assigned projection image of the target region image dataset (21) by forward projection from the reference dataset (18) and to compare the same with the assigned projection image in the projection space.

14. Computer program, which carries out the steps of a method according to one of claims 1 to 12 when it is executed on a control facility (17) of a computed tomography facility (1).

15. Electronically-readable data medium, on which a computer program according to claim 14 is stored.

## Revendications

1. Procédé de détermination d'une information de compensation pour la compensation d'une flexion d'une couchette (4) d'une table objet, en particulier d'une table (5) de patient, d'un dispositif (1) de tomodensitométrie assistée par ordinateur sous le poids d'un objet à recevoir, en particulier d'un patient (2), dans lequel on enregistre, par le dispositif (1) de tomodensitométrie assistée par ordinateur en une position d'enregistrement de la couchette (4), un ensemble (21) de données d'image d'une partie (3) cible de l'objet à examiner, dans lequel il est prévu, sur ou dans la couchette (4), au moins un marqueur (15) visible dans l'imagerie par rayons X et on se procure un ensemble (18) de données de référence en trois dimensions du marqueur (15), lorsque la couchette (4) n'est pas chargée, dans lequel on détermine l'information de compensation par une comparaison de l'ensemble (18) de données de référence à l'ensemble (21) de données d'image de la partie cible,
**caractérisé en ce que**, pour comparer l'ensemble (18) de données de référence à l'ensemble (21) de données d'image de la partie cible, on détermine, par projection en avant à partir de l'ensemble (18) de données de référence, au moins une image de comparaison ayant une géométrie d'enregistrement d'une image de projection associée de l'ensemble (21) de données d'image de la partie cible et on la compare dans la surface de projection à l'image de projection associé.

2. Procédé suivant la revendication 1, **caractérisé en ce que** l'on détermine, alors que la couchette (4) n'est pas chargée, l'ensemble (18) de données de référence dans une mesure de référence effectuée avec des propriétés de couchette, en particulier lors de la mise en fonctionnement pour la première fois du dispositif (1) de tomodensitométrie assistée par ordinateur en cours et/ou on se le procure à partir de données de construction de la table objet.

3. Procédé suivant la revendication 2, **caractérisé en ce que**, pour la mesure de référence, on sélectionne une position en hauteur de référence de la couchette (4) réglable en hauteur, de manière à détecter le au moins un marqueur (15) dans chaque image de projection de la mesure de référence, dans lequel l'ensemble (18) de données de référence est, pour la détermination de l'information de compensation lors d'un écart du réglage en hauteur à la position en hauteur de référence, décalé de cet écart dans la direction en hauteur.

4. Procédé suivant l'une des revendications précédentes, **caractérisé en ce que** l'on utilise, comme marqueur (15), au moins un élément (16) de marqueur oblong, en particulier de type en fil s'étendant dans une direction de déplacement de la couchette (4) parallèle, en particulier à un axe de rotation de la source (10) de rayons X du dispositif (1) de tomodensitométrie assistée par ordinateur, et/ou correspondant à une direction longitudinale de la couchette (4).

5. Procédé suivant la revendication 4, **caractérisé en ce que** l'élément (16) formant marqueur s'étend au moins sensiblement sur toute la longueur de la couchette (4) et/ou l'élément (16) formant marqueur est intégré à la couchette (4) et/ou on utilise au moins trois éléments (16) parallèles formant marqueur, en particulier un au milieu et deux, de préférence symétriquement, qui en sont voisins, de préférence dans les parties de bord de la couchette (4) et/ou à des niveaux différents dans la couchette (4).

6. Procédé suivant l'une des revendications précédentes, **caractérisé en ce que** l'on détermine l'ensemble (18) de données de référence en ne pointant que le au moins un marqueur (15).

7. Procédé suivant l'une des revendications précédentes, **caractérisé en ce que**, pour comparer l'ensemble (18) de données de référence à l'ensemble (21) de données d'image de la partie cible, on détermine, par projection en avant à partir de l'ensemble (18) de données de référence, pour plusieurs, de préférence pour toutes les images de projection pointant au moins l'un des au moins un marqueur (15) de l'ensemble (21) de données d'image de la partie cible, au moins une image de comparaison ayant une géométrie d'enregistrement de l'image de projection associée de l'ensemble (21) de données d'image de la partie cible et on la compare dans l'espace de projection à l'image de projection associée, dans lequel on détermine, pour chaque image de projection associée de l'ensemble de données d'image de la partie cible, plusieurs images de comparaison ayant des niveaux relatifs différents du au moins un marqueur, dans lequel on détermine, comme information de compensation, le niveau relatif ayant un coïncidence la plus grande de la au moins une image de comparaison correspondante à la au moins une image de projection associée.

8. Procédé suivant l'une des revendications précédentes, **caractérisé en ce que** l'on filtre passe haut les images de comparaison et les images de projection avant la comparaison en utilisant un même filtre.

9. Procédé suivant l'une des revendications précédentes, **caractérisé en ce que** l'on détermine, à partir de l'ensemble (18) de données de référence en tenant compte de la géométrie d'enregistrement pour l'ensemble (21) de données d'image de la partie cible des images de comparaison pour des niveaux relatifs différents du au moins un marqueur (15), dans lequel on détermine, comme information de compensation, un niveau relatif optimisé ayant une coïncidence la plus grande de la au moins une image de comparaison correspondante à au moins une image de la partie cible correspondante de l'ensemble (21) de données d'image de la partie cible.

10. Procédé suivant la revendication 9, **caractérisé en ce que** l'on choisit le niveau optimisé par un algorithme d'optimisation pour maximiser la coïncidence décrite, en particulier par une mesure de similitude et/ou de corrélation.

11. Procédé suivant l'une des revendications précédentes, **caractérisé en ce que**, pour la détermination de l'information de compensation, on utilise, comme données de sortie, une fonction entraînée sur la base de l'ensemble (18) de données de référence, utilisant au moins une partie de l'ensemble (21) de données d'image de partie cible comme données d'entrée.

12. Procédé suivant la revendication 11, **caractérisé en ce que** l'on produit, comme données d'entraînement pour la fonction entraînée, des ensembles de données d'entraînement, qui comprennent l'ensemble (18) de données de référence avec au moins un marqueur (15) disposé à des niveaux relatifs différents.

13. Dispositif (1) de tomodensitométrie assistée par ordinateur à utiliser dans des opérations, en particulier des interventions se rapportant à une partie (3) cible, en particulier d'un patient (2) comportant une table objet, en particulier une table (5) de patient ayant une couchette (4), déplaçable dans au moins une direction, pour l'objet, dans lequel il est prévu, sur ou dans la couchette (4), au moins un marqueur (15) visible dans l'imagerie par rayons X et un dispositif (17) de commande, qui a :
- un moyen (25) de mémoire, dans lequel un ensemble (18) de données de référence en trois dimensions du marqueur (15) est mis en mémoire, alors que la couchette (4) n'est pas chargée,
- une unité d'enregistrement, pour l'enregistrement en une position d'enregistrement de la couchette (4) d'un ensemble (21) de données de la partie cible pointant la partie (3) cible de l'objet,
- une unité (27) de détermination d'une information de compensation, pour la détermination d'une information de compensation pour la compensation d'une flexion de la couchette (4), à la position d'enregistrement sous le poids de l'objet à enregistrer, dans lequel l'unité (27) de détermination d'une information de compensation a une unité (28) de compensation pour la détermination de l'information de compensation, à partir d'une comparaison de l'ensemble (18) de données de référence à l'ensemble (21) de données d'image de la partie cible, et
- un dispositif (29) de compensation, pour la compensation d'une information de localisation, à utiliser pour l'opération et déterminée à partir de l'ensemble (21) de données d'image de la partie cible, en ce qui concerne la partie (3) cible, en particulier en ce qui concerne une position opératoire différente de la position d'enregistrement, sur la base de l'information de compensation,
- **caractérisé en ce que** le dispositif (1) de tomodensitométrie assistée par ordinateur est, pour la comparaison de l'ensemble (18) de données de référence à l'ensemble (21) de données d'image de la partie cible, agencé pour déterminer, par projection en avant à partir de l'ensemble (18) de données de référence, au moins une image de comparaison à une géométrie d'enregistrement d'une image de projection associée de l'ensemble (21) de données d'image de la partie cible et pour la comparer dans l'espace de projection à l'image de projection associée.

14. Programme d'ordinateur, qui effectue les stades d'un procédé suivant l'une des revendications 1 à 12, lorsqu'il est exécuté sur un dispositif (17) de commande d'un dispositif (1) de tomodensitométrie assistée par ordinateur.

15. Support de données, déchiffrable électroniquement, sur lequel un programme d'ordinateur suivant la revendication 14 est mis en mémoire.
